# EUROPEAN PATENT SPECIFICATION

(11) **EP 2 755 637 B1**
(45) Date of publication and mention of the grant of the patent: **12.08.2020**
(21) Application number: 12831341.8
(22) Date of filing: 13.09.2012
(51) Int. Cl.: A61K 9/14, A61K 47/34, A61K 31/366, A61K 31/397, A61K 31/4545

(54) **SOLID DISPERSION OF POORLY SOLUBLE COMPOUNDS COMPRISING CROSPOVIDONE AND AT LEAST ONE WATER-SOLUBLE POLYMER**
FESTE DISPERSION AUS SCHWERLÖSLICHEN VERBINDUNGEN MIT CROSPOVIDON UND MINDESTENS EINEM WASSERLÖSLICHEN POLYMER
DISPERSION SOLIDE DE COMPOSÉS PEU SOLUBLES COMPRENANT UNE CROSPOVIDONE ET AU MOINS UN POLYMÈRE HYDROSOLUBLE

(30) Priority: 13.09.2011 US 201161533835 P; 16.12.2011 US 201161576478 P
(43) Date of publication of application: 23.07.2014
(73) Proprietor: ISP Investments LLC, Wilmington, Delaware 19805 (US)
(72) Inventor: BI, Yunxia, Garnet Valley, PA 19060 (US); RAHMAN, Mohammed, Abdul, Columbia, MD 21045 (US); LESTER, James, David, Laurel, MD 20723 (US)
(74) Representative: Brosch, Oliver
(86) International application number: PCT/US2012/055129
(87) International publication number: WO 2013/040187

(56) References cited:
- EP-A1- 0 429 187
- EP-A2- 0 078 430
- EP-A2- 0 232 155
- GB-A- 2 139 892
- US-A- 5 266 581
- US-A1- 2008 181 961
- US-B1- 7 607 596

## Description

### FIELD

The present application relates to a solid dispersion composition, and, more particularly, a solid dispersion composition of poorly soluble compounds comprising crosslinked polyvinylpyrrolidone (crospovidone) and at least one water-soluble polymer.

### BACKGROUND

It has been estimated that more than 60% of Active Pharmaceutical Ingredients (API) in development have poor bioavailability due to low aqueous solubility (*Manufacturing chemist,* March 2010, 24-25). This percentage is likely to increase in the future with the increased use of combinatorial chemistry in drug discovery targeting lipophilic receptors. Poor bioavailability results in increased development times, decreased efficacy, increased *inter-* and *intra-* patient variability and side effects, and high dose that reduce patient compliance and increase cost. Thus, the ability to improve drug solubility and/ or dissolution rate and, hence, bioavailability through formulation technology is critical to improve a drug product's efficacy and safety, and reduce its cost. In recent years, one of the major focuses for the pharmaceutical formulators is to identify strategies that would improve the bioavailability of active pharmaceutical ingredients (APIs) by enhancing their dissolution rate and/or solubility. In particular, poorly soluble API's can be changed to amorphous or microcrystalline forms through formulation approaches, which provide a fast dissolution rate and/or higher apparent solubility in the gastric and intestinal fluids.

One of the approaches to address the problems related to poorly water soluble APIs is to formulate them as solid dispersions. Solid dispersion is a dispersion of active ingredients in an inert carrier or matrix at solid state prepared by the melting (fusion), solvent or melting-solvent method (J. Pharm. Sci., 1971; 60:1281-1302). It provides a drug release profile that is largely determined by the carrier properties.

At the early stage of solid dispersions development, small molecule crystalline carriers were used, wherein the solid dispersions of API were integrated in the crystal lattice of the carrier. A well known example of small molecule crystalline carrier is urea (Chem. Pharm. Bull 12, 134-144, 1964). The solid dispersion is a eutectic mixture that can increase the dissolution rate. However due to the crystalline nature of the poorly soluble APIs in the system; solubility enhancement cannot be expected, hence the bioavailability enhancement by such systems could be limited.

Later on, binary solid dispersions were developed with amorphous or crystalline polymeric carriers, in which API compounds were dispersed in amorphous form (Int. J. Pharm. 303, 132-142, 2005). Exemplary polymer carriers employed to prepare a binary solid dispersion include polyvinylpyrrolidone (Int. J. Pharm. 302, 103-112, 2005), polyethyleneglycols *(*J. Pharm. Sci. 59, 937-942, 1970), polymethacrylates (Int. J. Pharm. 319, 44-54, 2006), hydroxypropylmethylcellulose (J. Pharm. Sci. 95, 2692-2705, 2006), ethylcellulose (Int. J. Pharm. 327, 45-50, 2006), hydroxypropylcelluloses (J. Contr. Release 108, 386-395, 2005), and cyclodextrins (Eur. J. Pharm. Sci. 26, 184-193, 2005).

Although these binary solid dispersions resulted in significantly improved solubility of the API, challenges in developing formulations with desirable properties still remained. One such challenge is the physical instability of the API, *i.e.,* during processing or storage, the amorphous compounds tend to convert back to their crystalline forms. The most commonly used solid dispersion carriers are water soluble polymers. They can kinetically and/or thermodynamically stabilize these amorphous compounds and inhibit the recrystallization of the compound in the GI tract; however, for some compounds with very strong crystallization tendency, a water soluble polymer that can provide satisfactory stability and dissolution performance might not be found.

To overcome the above mentioned challenges, more functional excipients have been employed in solid dispersions. For example, solid dispersions may comprise a poorly water soluble API, an polymeric carrier and a suitable surface active agent to improve the dissolution profile. Such solid dispersions are developed to further increase the bioavailability of poorly water soluble APIs by overcoming API recrystallization. It is experienced that the addition of surfactant to the binary solid dispersion comprising an polymeric carrier helps to prevent precipitation and/or protect a fine crystalline precipitate from agglomeration into much larger hydrophobic particles. US Patent Publication No. 20110014282 discloses a solid dosage form for release of API, the solid dosage form comprising a solid dispersion, the solid dispersion comprising an active pharmaceutical ingredient belonging to BCS Class II, an amorphous carrier and a surfactant, wherein the amount of surfactant is from 0.5 to 30% of the total weight of the solid dispersion, and wherein at least 30% of the active pharmaceutical ingredient is in an amorphous form.

In another approach, a solid dispersion comprising API with two different polymers is employed. Particularly, JP Patent Application No. 2004-67606 discloses a tablet comprising fine granules obtained by spraying a solution containing itraconazole, which is a poorly soluble drug, a water-soluble polymer and an enteric polymer, on a mixed powder of an excipient and a disintegrator, granulating and drying. Karel Six et al. (J. Pharm. Sci. 93, 124-131, 2004) discloses a ternary solid dispersion composition of Itraconazole, a class II drug, Eudragit E100 and copovidone. The use of a combination of fast- and slow- dissolving polymers in solid dispersions compositions has resulted in increased physical stability and improved dissolution properties of itraconazole. In another approach, Hirasawa et al. (J. Pharm. Soc. of Japan, 124(1), 19-23, 2004; Chem. Pharm. Bull. 52(2) 244-247, 2004*;* JP Patent Application No. 2001335483A) disclose a solid dispersion comprising Nilvadipine (NIL)/ Crospovidone (cl-PVP)/ Methylcellulose (MC). US Patent Publication No. 20070248681 assigned to Shin-Etsu Chemical Co., Ltd. disclose a granule of a solid dispersion comprising a poorly soluble drug, a water-soluble polymer, an excipient and a disintegrator, wherein the content of the water-soluble polymer is 1 to 10% by weight and the content of the disintegrator is 15 to 50% by weight. A method for producing a tablet of a solid dispersion is also disclosed.

In the present application, some of the limitations set forth above are addressed by a new ternary solid dispersion composition comprising (a) at least one poorly soluble API, (b) at least one water-soluble polymer, and (c) crospovidone, a water-insoluble polymer. Surprisingly, the ternary solid dispersion composition demonstrates superior physical stability upon storage, and/or inhibits recrystallization of API's in bio-relevant media more efficiently than binary solid dispersion compositions. Further, this unique ternary system with crospovidone is capable of providing significantly enhanced bioavailability because of superior dissolution of the API resulting from availability of high surface area, high interfacial activity and particle morphology of water-insoluble crospovidone.

**Further reference can be made to** EP 0 429 187 (A1**) disclosing formulations comprising an adsorbate of a mixture of a pharmaceutically useful steroid and a polyvinylpyrrolidone adsorbed on a cross-linked polyvinylpyrrolidone.**

### SUMMARY

The present application provides a stable ternary solid dispersion composition with enhanced bioavailability comprising: (a) 1% *wt*. to 50% *wt.* of one or more poorly soluble active pharmaceutical ingredient (API) which belong to Biopharmaceutics Classification System (BCS) class II and/or IV; (b) 11% *wt.* to 50% *wt.* of at least one water-soluble polymer and; (c) 20% *wt.* to 99% *wt.* of crosslinked polyvinylpyrrolidone (crospovidone, a water-insoluble polymer); and wherein the solid dispersion is capable of inhibiting crystallization of API in solid state and/or biorelavent aqueous medium.

The present application provides a method for preparing the solid dispersion composition by hot-melt **extrusion.**

**A reference process** for preparing a solid dispersion composition **comprises the steps of:** (a) preparing a homogenous aqueous and/or organic solution of (i) one or more water-soluble polymers, and (ii) at least one active pharmaceutical ingredient (API) which belongs to BCS class II and/or IV; (b) suspending crosslinked polyvinylpyrrolidone (crospovidone, a water-insoluble polymer) in the resultant homogenous aqueous and/or organic solution of step (a) to yield a suspension or dispersion; and (c) spray-drying the resultant of step (b) to yield a dry powder form of a solid dispersion composition.

In accordance with the present application, there is provided a process for preparing a solid dispersion composition comprising the steps of: (a) preparing a homogenous blend of (i) at least one active pharmaceutical ingredient (API) which belongs to BCS class II and/or IV; (ii) one or more water-soluble polymer; and (iii) crosslinked polyvinylpyrrolidone (crospovidone, a water-insoluble polymer); (b) heating, mixing and/or kneading the resultant blend of step (a) through an extruder to result in a homogenous melt and/or granulation; (c) forcing the resultant melt obtained in step (b) through one or more orifices, nozzles, or moulds; (d) cooling the extrudate of step (c) by means of air to yield a solid dispersion; and (e) optionally, grinding the solid dispersion obtained in step (d).

In yet another aspect of the present application, the desired solid dispersion composition further comprises a pharmaceutically acceptable excipient selected from the group consisting of surfactants, plasticizers, disintegrants, lubricants, glidants, carriers, anti-adherents, inert fillers, wetting agents, pH modifiers, binders, solubility modifiers, recrystallization inhibitors, alone or in combination.

### DETAILED DESCRIPTION

While this specification concludes with claims particularly pointing out and distinctly claiming that which is regarded as the invention, it is anticipated that the invention can be more readily understood through reading the following detailed description of the invention and study of the included examples.

The singular forms "a," "an," and "the" include plural forms unless the context clearly dictates otherwise specified or clearly implied to the contrary by the context in which the reference is made. The term "comprising" and "comprises of includes the more restrictive claims such as "consisting essentially of" and "consisting of".

The term "about" can indicate a difference of 10 percent of the value specified. Numerical ranges as used herein are meant to include every number and subset of numbers enclosed within that range, whether particularly disclosed or not. Further, these numerical ranges should be construed as providing support for a claim directed to any number or subset of numbers in that range.

All percentages, parts, proportions and ratios as used herein, are by weight of the total composition, unless otherwise specified. All such weights as they pertain to listed ingredients are based on the active level and, therefore; do not include solvents or byproducts that may be included in commercially available materials, unless otherwise specified.

All references to singular characteristics or limitations of the present invention shall include the corresponding plural characteristic or limitation, and *vice-versa,* unless otherwise specified or clearly implied to the contrary by the context in which the reference is made.

As used herein, the words "preferred" or "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

References herein to "one embodiment" or "one aspect" or "one version" or "one objective" of the invention include one or more such embodiment, aspect, version or objective, unless the context clearly dictates otherwise.

The term "poorly soluble" refers to slightly soluble or very slightly soluble compounds and it requires from about 100 or more parts of solvent for one part of solute and wherein the poorly soluble means the solubilization of API compounds becomes the rate limiting step for absorption of such API compounds.

The term "polymer" refers to a compound comprising repeating structural units (monomers) connected by covalent chemical bonds. Polymers may be further derivatized, crosslinked, grafted or end-capped. Non-limiting examples of polymers include copolymers, terpolymers, quaternary polymers, and homologues. The term "copolymer" refers to a polymer consisting essentially of two or more different types of repeating structural units (monomers).

According to the present application, the term "solid dispersion" means a solid state which comprises at least two constituents, wherein one constituent is homogenously dispersed significantly evenly throughout the other constituent or constituents. It includes solid or glassy solutions, *i.e.,* the dispersion of the constituents is in such a way that the composition is chemically and physically homogenous in nature. The "solid dispersion" of the present application advantageously comprises a co-precipitate or co-melt of an active pharmaceutical ingredient (API), crosslinked PVP and at least one or more water-soluble polymers, wherein the API is dispersed significantly uniformly all over the carrier system. The API may be present in an amorphous state or in fine crystalline dispersed form. Also, the API may be available as a mixture of amorphous and crystalline forms.

The term "Pharmaceutically acceptable excipient" refers to an inert additive included to solid formulations in the form such as powders, granules, capsules, pellets and tablets to increase the bulk of the desired formulation comprising the present solid dispersion. The excipients may be added during or after the preparation of hot-melt or spray-dried form of the solid dispersion composition.

What is described herein is a stable ternary solid dispersion composition with enhanced bioavailability comprising: (a) 1% *wt*. to 50% *wt.* of one or more poorly soluble active pharmaceutical ingredients (APIs) which belongs to Biopharmaceutics Classification System (BCS) class II and/or IV; (b) 11% *wt.* to 50% *wt.* of at least one water-soluble polymer; and (c) 20% wt. to 99% *wt.* of crosslinked polyvinylpyrrolidone (crospovidone, a water-insoluble polymer); and wherein the solid dispersion is capable of inhibiting crystallization and or crystal growth of API in solid state and/or aqueous gastrointestinal tract (GIT) medium.

According to one embodiment of the present application, the poorly soluble drugs are BCS Class II drugs having high permeability and low solubility; or Class IV drugs having low permeability and low solubility. The BCS Class II or Class IV API may belong to analgesics, anti-inflammatory agents, anti-helminthics, anti-arrhythmic agents, anti-bacterial agents, anti-viral agents, anti-coagulants, anti-depressants, anti-diabetics, anti-epileptics, antifungal agents, anti-gout agents, anti-hypertensive agents, anti-malarials, anti-migraine agents, anti-muscarinic agents, anti-neoplastic agents, erectile dysfunction improvement agents, immune-suppressants, anti-protozoal agents, anti-thyroid agents, anxiolytic agents, sedatives, hypnotics, neuroleptics, β-blockers, cardiac inotropic agents, corticosteroids, diuretics, anti-parkinsonian agents, gastro-intestinal agents, histamine receptor antagonists, keratolyptics, lipid regulating agents, anti-anginal agents, Cox-2 inhibitors, leukotriene inhibitors, macrolides, muscle relaxants, nutritional agents, opiod analgesics, protease inhibitors, sex hormones, stimulants, muscle relaxants, anti-osteoporosis agents, anti-obesity agents, cognition enhancers, anti-urinary incontinence agents, nutritional oils, anti-benign prostate hypertrophy agents, essential fatty acids, non-essential fatty acids, antipyretics, muscular relaxants, anti-convulsants, anti-emetics, anti-psychotics, and/or anti-Alzheimer agents.

The APIs that belong to BCS Class II are poorly soluble, but are absorbed from the solution by the lining of the stomach and/or intestine.

The non-limiting BCS Class II drugs are selected from the group consisting of Albendazole, Acyclovir, Azithromycin, Cefdinir, Cefuroxime axetil, Chloroquine, Clarithromycin, Clofazimine, Diloxanide, Efavirenz, Fluconazole, Griseofulvin, Indinavir, Itraconazole, Ketoconalzole, Lopinavir, Mebendazole, Nelfinavir, Nevirapine, Niclosamide, Praziquantel, Pyrantel, Pyrimethamine, Quinine, Ritonavir, Bicalutamide, Cyproterone, Gefitinib, Imatinib, Tamoxifen, Cyclosporine, Mycophenolate mofetil, Tacrolimus. Acetazolamide, Atorvastatin, Benidipine, Candesartan cilexetil, Carvedilol, Cilostazol, Clopidogrel, Ethylicosapentate, Ezetimibe, Fenofibrate, Irbesartan, Manidipine, Nifedipine, Nisoldipine, Simvastatin, Spironolactone, Telmisartan, Ticlopidine, Valsartan, Verapamil, Warfarin, Acetaminophen, Amisulpride, Aripiprazole, Carbamazepine, Celecoxib, Chlorpromazine, Clozapine, Diazepam, Diclofenac, Flurbiprofen, Haloperidol, Ibuprofen, Ketoprofen, Lamotrigine, Levodopa, Lorazepam, Meloxicam, Metaxalone, Methylphenidate, Metoclopramide, Nicergoline, Naproxen, Olanzapine, Oxcarbazepine, Phenyloin, Quetiapine, Risperidone, Rofecoxib, Valproic acid, Isotretinoin, Dexamethasone, Danazol, Epalrestat, Gliclazide, Glimepiride, Glipizide, Glyburide (glibenclamide), levothyroxine sodium, Medroxyprogesterone, Pioglitazone, Raloxifene, Mosapride, Orlistat, Cisapride, Rebamipide, Sulfasalazine, Teprenone, Ursodeoxycholic Acid, Ebastine, Hydroxyzine, Loratadine, and Pranlukast.

The non-limiting BCS Class IV drugs are selected from the group consisting of acetazolamide, furosemide, tobramycin, cefuroxmine, allopurinol, dapsone, doxycycline, paracetamol, nalidixic acid, clorothiazide, tobramycin, cyclosporin, tacrolimus, paclitaxel, prostaglandines, prostaglandine E2, prostaglandine F2, prostaglandine E1, proteinase inhibitors, indinavire, nelfinavire, saquinavir, cytotoxics, doxorubicine, daunorubicine, epirubicine, idarubicine, zorubicine, mitoxantrone, amsacrine, vinblastine, vincristine, vindesine, dactiomycine, bleomycine, metallocenes, titanium metallocene dichloride, lipiddrug conjugates, diminazene stearate, diminazene oleate, chloroquine, mefloquine, primaquine, vancomycin, vecuronium, pentamidine, metronidazole, nimorazole, tinidazole, atovaquone, buparvaquone.

The above disclosed non-limiting BCS Class II and IV drugs can be a free acid, free base or neutral molecules, or in the form of an appropriate pharmaceutically acceptable salt, a pharmaceutically acceptable solvate, a pharmaceutically acceptable co-crystal, a pharmaceutically acceptable enantiomer, a pharmaceutically acceptable derivative, a pharmaceutically acceptable polymorph, pharmaceutically acceptable ester, pharmaceutically acceptable amide or a pharmaceutically acceptable prodrug thereof.

According to another embodiment, the water-soluble polymer is selected from the group consisting of an acid, ester, amide or salts of olefinic polymers, lactam/pyrrolidone based polymers, pyrrolidone co-polymers, cellulose based polymers, cellulose based copolymers, α-olefin maleic acid/ester co-polymers, α-olefin polymers, carbohydrate based polymers, natural polymers, or gums alone or in combination.

Water-soluble polymers useful in the present application include those capable of forming a desired solid dispersion composition. The preferred water-soluble polymers are homopolymers of *N*-vinyllactam, copolymers of *N*-vinyllactam, homopolymers of vinylpyrrolidone, copolymers of vinylpyrrolidone, cellulose esters, cellulose ethers, polyalkylene oxides, polyacrylates, polymethacrylates, homo and co-polymers of acrylic acids, homo and co-polymers of methacrylic acids, co-polyacrylamides, polyvinyl alcohols, vinyl acetate polymers, co-polymers or vinylacetate, carboxyvinyl polymers, oligosaccharides, and polysaccharides.

Examples of typical water-soluble polymer species include, but are not limited to the following categories, *i.e.,* Lactam/Pyrrolidone based polymers, Polyvinyl pyrrolidone/polyvinyl caprolactam, Pyrrolidone co-polymers, Vinyl acetate-Vinylpyrrolidone co-polymers, Alkylated graft Vinylpyrrolidone co-polymers, Dimethylaminoethylmethacrylate, Vinylpyrrolidone co-polymers, Acrylic acid/ Acrylic ester/ Acrylic salt - Vinylpyrrolidone co-polymers, Vinylpyrrolidone/Vinyl caprolactam co-polymers, Alpha olefin maleic acid/ester co-polymers, Styrene maleic acid co-polymers, Alkyl vinyl ether-maleic acid/ester/salts co-polymers, Alpha olefin Polymers: Polyacrylates/ polyvinyl derivatives, Poly alkylacrylate/alkylacrylic esters/amides/salts, Polyvinyl alcohol/acetates, Natural polymers, Cellulosic derivatives, Modified Starch and/or alginates.

The water-soluble polymer is employed in an amount sufficient to form a desired composition. The water-soluble polymer will be present in an amount by weight percent of from 15% *wt.* to 35% *wt.*

According to one important embodiment of the present application, the ternary solid dispersion composition comprises a crosslinked polyvinylpyrrolidone (crospovidone), a water-insoluble synthetic homopolymer of N-vinyl-2-pyrrolidone. Chemically the repeat structure of crospovidone is similar to N-methylpyrrolidone (NMP), a water-miscible, polar aprotic solvent with high interfacial activity used as a solubilizer in many applications. Crospovidone particles are granular and porous with popcorn-like morphology. This particle morphology and the small particle size of crospovidone provide high surface area. The high surface area combined with unique chemistry results in high interfacial activity that serves to enhance the dissolution of poorly soluble drugs. When used in combination with water soluble polymers in solid dispersion formulations, crospovidone can further enhance the physical stability of APIs in the formulation, and/or prolong the duration of supersaturation in bio-relevant dissolution media/GI tract *via* the following non-limiting mechanisms (i) forming non-specific and/or specific interaction for example, hydrogen-bonding with APIs enhances the physical stability of APIs thermodynamically and/or kinetically; and/or (ii) due to the porous structure and insoluble nature of crospovidone, the API could be embedded in the porous structure as amorphous and/or nano crystalline particles. The physical barriers provided by crospovidone can reduce the mobility of API molecules and hence the rates of nucleation and crystal growth, and therefore the physical stability of APIs can be further enhanced kinetically. For these reasons, it is believed that a water-insoluble crospovidone enhances the bioavailability of poorly insoluble API's.

The range of crospovidone is the range of from 40% wt. to 60% wt.

Commercial PVPP products are sold into commercial sale by a number of manufacturers/suppliers. Polyplasdone® and Polyclar® grades are available from Ashland Specialty Ingredients (Wilmington, DE). Three grades comprise the Polyplasdone® product line: INF-10, XL, and XL-10. PVPP is offered for sale by BASF Corp. (Ludwigshafen, DE) in the Kollidon® CL and Luvicross® product lines. Four grades of pharmaceutical-grade crospovidone compose the Kollidon® CL line: CL, CL-M, CL-F, and CL-SF. The industrial grade of PVPP is sold under the Luvicross® name. In addition, PVPP of the Sunvidone® CL series is sold by Hangzhou Sunflower Technology Development Co. (Hangzhou, CN). Three grades are available, CL-10, CL-30, and CL-100.

It is an optional embodiment of the present application to use other suitable water-insoluble polymers for preparation of a solid dispersion, such polymers can be selected from the group including but not limited to oils, beeswax, carnauba wax, microcrystalline wax, fatty alcohols, cetostearyl alcohol, stearyl alcohol, cetyl alcohol, myristyl alcohol, fatty acid esters, glyceryl monostearate, glycerol distearate, glycerol monooleate, acetylated monoglycerides, tristearin, tripalmitin, cetyl esters wax, glyceryl palmitostearate, glyceryl behenate, celluloses, ethylcellulose, low substituted hydroxypropyl cellulose (L-HPC), cellulose acylate, cellulose diacylate, cellulose triacylate, cellulose acetate, cellulose diacetate, cellulose triacetate, mono cellulose alkanylates, di cellulose alkanylates tri cellulose alkanylates, mono cellulose arylates, di cellulose arylates, tricellulose arylates, mono cellulose alkenylates, di cellulose alkenylates, tri cellulose alkenylates, ethyl acrylate-methyl methacrylate-trimethylammonioethyl methacrylate chloride copolymer, methacrylic acid-ethyl acrylate copolymer, methacrylic acid-methyl methacrylate copolymer, carboxymethylcellulose sodium (carmellose sodium), croscarmellose sodium, acacia, tragacanth, propylene glycol alginate, agar powder, gelatin, starch, partly pregelatinized starch, oil, sodium starch glycolate, phospholipid (lecithin), glucomannans, polymethacrylic acid based polymers, zein, aliphatic polyesters, or mixtures of any two or more in various ratios and proportions as required without limitation.

The ratio of Active Pharmaceutical Ingredient (API, a) to water-soluble polymer (b) to crospovidone (c) may be from about 0.1- 3: 0.1- 3: 1- 5. The preferred ratio is from about 0.5-1.5: 0.5-1.5: 1.5-2.5 and the most preferred ratio is 1: 1: 2.

Suitable surfactant or surfactant system for preparing solid dispersion composition of the present application can be selected from anionic, non-ionic, amphoteric, cationic and mixtures thereof. The contemplated list of surfactants for the present application is as follows:
(A) **Anionic Surfactants:** Anionic surfactants are particularly useful in accordance with certain embodiments of the present application. Surfactants of the anionic type that may be useful include:
   (1) Sulfonates and Sulfates: Suitable anionic surfactants include sulfonates and sulfates such as alkyl sulfates, alkylether sulfates, alkyl sulfonates, alkylether sulfonates, alkylbenzene sufonates, alkylbenzene ether sulfates, alkylsulfoacetates, secondary alkane sulfonates, secondary alkylsulfates, alkyl sulfosuccinates and the like. Further, examples of anionic surfactants include water-soluble salts of higher fatty acid monoglyceride monosulfates, such as the sodium salt of the monosulfated monoglyceride of hydrogenated coconut oil fatty acids, higher alkyl sulfates such as sodium lauryl sulfate, alkyl aryl sulfonates such as sodium dodecyl benzene sulfonate, higher alkyl sulfoacetates, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like.
   (2) Phosphates and Phosponates: Suitable anionic surfactants also include phosphates such as alkyl phosphates, alkylether phosphates, aralkylphosphates, and aralkylether phosphates. Examples include a mixture of mono-, di- and tri-(alkyltetraglycolether)-*o*-phosphoric acid esters generally referred to as trilaureth-4-phosphate commercially available under the trade designation HOSTAPHAT 340KL from Clariant Corp., as well as PPG-5 ceteth 10 phosphate available under the trade designation CRODAPHOS SG from Croda Inc., Parsipanny, NJ.
   (3) Amine Oxides: Suitable anionic surfactants also include amine oxides. Examples of amine oxide surfactants include lauryldimethylamine oxide, laurylamidopropyldimethylamine oxide, and/or cetyl amine oxide.
**(B) Amphoteric Surfactants:** Surfactants of the amphoteric type include surfactants having tertiary amine groups which may be protonated as well as quaternary amine containing zwitterionic surfactants. Those that may be useful include:
   (1) Ammonium Carboxylate Amphoterics: Examples of such amphoteric surfactants include, but are not limited to: certain betaines such as cocobetaine and cocamidopropyl betaine; monoacetates such as sodium lauroamphoacetate; diacetates such as disodium lauroamphoacetate; amino- and alkylamino-propionates such as lauraminopropionic acid.
   (2) Ammonium Sulfonate Amphoterics: This class of amphoteric surfactants are often referred to as "sultaines" or "sulfobetaines" for example, cocamidopropylhydroxysultaine.
(C) **Nonionic Surfactants:** Surfactants of the nonionic type that may be particularly useful include:
   (1) Polyethylene oxide extended sorbitan monoalkylates (i.e., Polysorbates); (2) Polyalkoxylated alkanols; (3) Polyalkoxylated alkylphenols include polyethoxylated octyl or nonyl phenols having HLB values of at least about 14, which are commercially available under the trade designations ICONOL and TRITON; (4) Polaxamers. Surfactants based on block copolymers of ethylene oxide (EO) and propylene oxide (PO) may also be effective. Both EO-PO-EO blocks and PO-EO-PO blocks are expected to work well as long as the HLB is at least about 14, and preferably at least about 16. Such surfactants are commercially available under the trade designations PLURONIC and TETRONIC from BASF; (5) Polyalkoxylated esters - Polyalkoxylated glycols such as ethylene glycol, propylene glycol, glycerol, and the like may be partially or completely esterified, *i.e.,* one or more alcohols may be esterified, with a (C₈ to C₂₂) alkyl carboxylic acid. Such polyethoxylated esters having an HLB of at least about 14, and preferably at least about 16, may be suitable for use in compositions of the present invention; (6) Alkyl Polyglucosides - This includes glucopon 425, which has a (C₈ to C₁₆) alkyl chain length with an average chain length of 10.3 carbons and 14 glucose units.
(D) **Cationic Surfactants:** Surfactants of the cationic type that may be useful include but are not limited to, primary amines, secondary amines, tertiary amines, quaternary amines, alkanolamines, mono-alkyl alkanolamines, di-alkyl alkanolamines, tri-alkyl alkanolamines, alkyl mono alkanolamines, alkyl di-alkanolamines, alkylamines, mono-alkyl amines, di-alkyl amines, tri-alkylamines, alkoxylated amines, alkyl and aryl amine alkoxylates, methoxylated alkylamines, ethoxylated alkylamines, alkoxylated alkanolamines, alkyl alkanolamines, alkoxylated ethylene diamine derivatives, alkyl/aryl/arylalkyl amine oxides. Preferred cationic surfactants of the present invention include, but are not limited to, (a) alkyl alkanolamines; and (b) alkyl tertiary amines. Additional information on useful cationic surfactants for the purpose of present invention is set forth in McCutcheon's Detergents and Emulsifiers, North American Ed., 1982 and Kirk-Othmer, Encyclopedia of Chemical Technology, 3rd Ed., Vol. 22, pp. 346-387.

Particularly, the preferred surfactants include dodecanesulfonic acid, sodium dodecyl sulfate, sodium lauryl sulfate (SLS), (poly)-oxyethylene sorbitan long-chain fatty acid esters, Vitamin E-TPGS, bile salts, sodium deoxycholate, sodium glycocholate and/or polyoxyethylene polyoxypropylene glycols. If desired, combinations of various surfactants can be used for the preparation of ternary solid dispersion composition.

The suitable plasticizers employed in the present application include by way of example and without limitation, acetyl triethyl citrate, acetyl tributyl citrate, triethyl citrate, acetylated monoglycerides, glycerol, polyethylene glycol, triacetin, propylene glycol, dibutyl phthalate, diethyl phthalate, isopropyl phthalate, dimethyl phthalate, dibutyl sebacate, dimethyl sebacate, castor oil, glycerol monostearate, fractionated coconut oil, low molecular weight polymers, oligomers, copolymers, oils, small organic molecules, low molecular weight polyols having aliphatic hydroxyls, ester-type plasticizers, glycol esters, poly(propylene glycol), multi-block polymers, single-block polymers, low molecular weight poly(ethylene glycol), citrate ester-type plasticizers, triacetin, propylene glycol, glycerin, ethylene glycol, 1,2-butylene glycol, 2,3-butylene glycol, styrene glycol, diethylene glycol, triethylene glycol, tetraethylene glycol and other poly(ethylene glycol) compounds, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, dibutylsebacate, acetyltributylcitrate, triethyl citrate, acetyl triethyl citrate, tributyl citrate, polyethylene glycols such as PEG 200, PEG 300, PEG 400 and PEG 600, and/or allyl glycolate.

According to one embodiment of the present application, at least one disintegrating agent can be added to the solid dispersion composition to facilitate the breakup or disintegration of a formulation when contacted with gastrointestinal (GIT) fluid. The suitable disintegrant can be selected from the group including, but not limited to, calcium carbonate, methylcellulose, cross-linked carboxymethylcellulose, cross-linked sodium carboxymethylcellulose, calcium carboxymethyl cellulose, low-substituted hydroxypropyl cellulose, carmellose, carmellose sodium, carmellose calcium, croscarmellose sodium, agar, guar, locust bean, karaya, pectin, tragacanth, bentonite, cation-exchange resin, polyvinylpyrrolidone, crosslinked polyvinyl pyrrolidone, alginic acid, alginates, sodium alginate, microcrystalline cellulose, polacrillin potassium, starch, pregelatinized starch, carboxymethyl starch, corn starch, potato starch, sodium starch glycolate, citrus pulp, sodium lauryl sulfate, sodium lauryl sulfate alone or in combination.

Lubricants and Glidants can be employed in the present application to prevent, reduce or inhibit adhesion or friction of ingredients of the composition. They facilitate the compression and ejection of compressed compositions from a desired die. They are compatible with the ingredients of the pharmaceutical composition, and they do not significantly reduce the solubility, hardness, chemical stability, physical stability, or the biological activity of the pharmaceutical composition. The pharmaceutically acceptable lubricants and glidants for the present application are selected from the group including but not limited to stearic acid, metallic stearates, zinc stearate, magnesium stearate, magnesium trisilicate, calcium hydroxide, tribasic calcium phosphate, magnesium carbonate, magnesium oxide, calcium stearate, glyceryl monostearate, waxes, glycerides, glyceryl behenate, glyceryl palmitostearate, silicone oil, hydrogenated vegetable oil, hydrogenated castor oil, light mineral oil, mineral oil, polyethylene glycol, methoxypolyethylene glycol, sodium acetate, sodium oleate, sodium chloride, leucine, sodium benzoate, alkyl sulfates, sodium lauryl sulfate, sodium stearyl fumarate, talc, colloidal silica, corn starch, powdered cellulose, and/or boric acid. The preferred range of lubricants/glidants is from about 0.5% to 10% of the composition.

According to one embodiment of the present application, suitable binders can be selected from the group including, but not limited to, starches, modified starches, pregelatinized starch, partially pregelatinized starch, agar, gelatin, dextrin, alginic acid, sodium alginate, agar, calcium carrageenan, tragacanth gum, xanthan gum, gum acacia, sugars, lactose, liquid glucose, guar gum, hyaluronic acid, pectin, wax binders, sodium chondroitin sulfate, polyvinylpyrrolidone, povidone, polyvinyl alcohol, carboxyvinyl polymer, polyacrylic acid-series polymer, polylactic acid, polyethylene glycol, cellulose ethers, methyl cellulose, ethyl cellulose, carboxymethyl cellulose, carboxymethyl cellulose sodium, hydroxyethyl cellulose, hydroxypropyl cellulose, hydroxypropyl methyl cellulose, microcrystalline cellulose, and/or polyethylene oxides.

The desired pH of the composition can be achieved by employing any suitable pH modifying agents. The non-limiting pH modifiers include weak carboxylic acids, citric acid, acetic add, lactic acid, tartaric acid, aspartic acid, succinic acid, phosphoric acid, salicylic acid, sulfamic acid, benzoic acid or their salts, phosphates, pyrophosphate and its salts, metaphosphate and its salts, carbonic acid and its salts, hydroxylammonium, adidic acid and its salts, maleic acid and its salts, ascorbic acid and its salts, sodium acetate, potassium acetate, calcium oxide, magnesium oxide, trisodium phosphate, sodium hydroxide, ammonium hydroxide, pottasium hydroxide, calcium hydroxide, aluminum hydroxide, amines, triethanol amine, diethanol amine, monoethanol amine.

Diluents or Filling agents increase the bulk of the composition to ease compression or produce sufficient bulk for homogenous blend for the composition. The appropriate diluents for the present application may be selected from the following non-limiting examples including lactose, anhydrous lactose, spray-dried lactose, mannitol, sorbitol, compressible sugar, starch, sucrose, dextrose, trehalose, maltose, xylitol, lactitol, amylase, calcium sulfate, calcium sulfate dehydrate, calcium lactate trihydrate, monobasic calcium sulfate monohydrate, calcium carbonate, tribasic calcium phosphate, diabasic calcium phosphate, maltodextrin, starch, modified starch, starch hydrolyzates, pregelatinized starch, microcrystalline starches, microcrystalline cellulose, powdered cellulose, cellulose and cellulose derivatives, hydroxypropylmethylcellulose, hydroxypropylmethylcellulose acetate stearate, glycine, kaolin, sodium chloride, inositol, bentonite.

**A reference** process for preparing a solid dispersion composition **comprises the steps of: a) preparing a homogenous aqueous and/or** organic solution of (i) one or more water-soluble polymer, and (ii) at least one active pharmaceutical ingredient (API) which belongs to BCS class II and/or IV; (b) suspending crosslinked polyvinylpyrrolidone (crospovidone), a water-insoluble polymer in the resultant homogenous aqueous solution of step (a) to yield a suspension or dispersion; and (c) spray-drying the resultant of step (b) to yield a dry powder form of solid dispersion composition.

The appropriate solvents for the preparation of solid dispersion should have significantly low toxicity and readily be removed from the solid dispersion to a level that is acceptable as per the International Committee on Harmonization (ICH) guidelines. The preferred solvents comprise alcohols, methanol, ethanol, n-propanol, iso-propanol, butanol, ketones, acetone, methyl ethyl ketone, methyl iso-butyl ketone, esters, ethyl acetate, butyl acetate, propylacetate, nitriles, acetonitrile, hydrocarbons, hexane, cyclohexane, toluene, halogenated hydrocarbons, methylene chloride, 1,1,1-trichloroethane, chloroform, ethers, cylic ethers, tetrahydrofuran, dioxane, ethyl ether, propyl ether, amides, dimethyl acetamide, dimethylsulfoxide, dimethylformamide, cellosolve, ethyl cellosolve, cellosolve acetate, methylcarbitol, N-methylpyrrolidone, organic acids, acetic acid alone or in combination. If required, pharmaceutically acceptable grade of water can be mixed with the desired organic solvent without disturbing the solubility of the API. The preferred solvents or mixture of solvents employed in the present application comprises Acetone:Water (9:1), Benzene:Methanol (1:1), Glycerin:Water (3:7), *t*-Butanol:Water (9:1), Toluene:Ethanol (3:2), THF:Methanol (2:1), Acetone:Methanol (2:1), Dichloromethane:Methanol (2:1), Chloroform, Cyclohexanone, Dimethyl formamide, Dimethyl sulfoxide, Dioxane, Ethylene chlorohydrin, Formic acid (88%), Tetrahydrofuran (THF).

The solvent can be eliminated by spray-drying through the techniques that are known in the prior art for a person skilled in the art. Moreover, the spray-drying processes and spray-drying equipment are described in detail in Perry's Chemical Engineers' Handbook, pages 20-54 to 20-57 (6th Edn. 1984). More details on spray-drying processes and equipment are reviewed by Marshall, "Atomization and Spray-Drying," 50 Chem. Eng. Prog. Monogr. Series 2 (1954), and Masters, Spray Drying Handbook (4th Edn. 1985). The driving force for solvent elimination or evaporation is usually provided by keeping the partial pressure of solvent in the spray-drying equipment substantially below the vapor pressure of the solvent at the temperature of the drying droplets.

The application relates to a method for preparing a solid dispersion composition comprising the steps of (a) preparing a homogenous blend of (i) at least one active pharmaceutical ingredient (API) which belongs to BCS class II and/or IV; (ii) one or more water-soluble polymer; and (iii) crosslinked polyvinylpyrrolidone (crospovidone), a water-insoluble polymer; (b) heating, mixing and kneading the resultant blend of step (a) through screw extruder to result in homogenous melt; (c) forcing the resultant melt obtained in step (b) through one or more orifices, nozzles, or moulds; (d) cooling the extrudate of step (c) by means of air to yield solid dispersion; and (e) optionally, grinding the solid dispersion obtained in step (d).

According to one embodiment of the present application, the mixture of poorly soluble API, water-soluble polymers and crospovidone is heated at a temperature wherein all the components of the mixture are melted, or a temperature wherein some components are melted. Hot-melt extrusion refers to a transition of components into a liquid or rubbery stage by heating in which one or more components get embedded homogenously in the other component. The preparation of a hot-melt extrusion comprises mixing together a desired API, at least one water-soluble polymer, and crospovidone until a soft mass is produced. Alternatively, the mixing of the desired API, water-soluble polymers and crospovidone can happen before, during or after the formation of the soft mass. For example, the required ingredients to produce the soft mass may be mixed initially and then extruded, or may be simultaneously mixed and melt extruded. Finally, the hot-melt is homogenized so as to disperse or embed the desired API into other ingredients or components.

The process of hot-melt extrusion may be carried out by using conventional extruders that are known in the art. The suitable extruders include, but not limited to, single screw extruders, intermeshing screw extruders or else multiscrew extruders, preferably twin screw extruders, which can be co-rotating or counter-rotating and, optionally, be equipped with kneading mixing and/or conveying elements. The working temperature for preparing hot-melt extrusion typically depends on the API and polymer properties as well as extruder type and screw configuration.

The extrudate obtained from extrusion may be in the form of beads, granulates, tube, strand, or cylinder and this can be further processed into any suitable shape. The term "extrudate" herein refers to glass solutions and amorphous and crystalline solid dispersions, and of one or more API, at least one water-soluble polymer, crospovidone and optionally one or more pharmaceutically acceptable excipients.

**Alternative** methods for preparing solid dispersions would include, but are not limited to, fusion/melt technology, hot-melt coating, prilling, melt-congealing, melt-granulation, spray-congealing, solvent-evaporation, spray-drying, co-precipitation, supercritical fluid method, and electrostatic spinning method.

According to one particularly preferred embodiment of this application, Loratadine (an API), Copovidone (a soluble polymer) and Crospovidone (a water-insoluble polymer) are melt extruded and milled by the process as described herein, to produce a powder blend of Loratadine, Copovidone, Crospovidone and optionally, one or more pharmaceutically acceptable excipients which may comprise suitable processing aids, bulking agents, plasticizer, surfactants, plasticizers, disintegrants, lubricants, glidants, carriers, anti-adherents, inert fillers, wetting agents, pH modifiers, binders, solubility modifiers, and/or recrystallization inhibitors.

According to one **reference** embodiment of this application, Loratadine, Copovidone (a soluble polymer) and Crospovidone (a water-insoluble polymer) are spray-dried by the process as described herein, to produce a spray-dried powder blend of Loratadine, Copovidone, Crospovidone and optionally, one or more pharmaceutical acceptable excipients, which may comprise suitable processing aids, bulking agents, plasticizer, surfactants, plasticizers, disintegrants, lubricants, glidants, carriers, anti-adherents, inert fillers, wetting agents, pH modifiers, binders, solubility modifiers, and/or recrystallization inhibitors.

According to one **reference** embodiment of this application, an API, particularly Lovastatin or Ezetimibe or Piroxicam, a water soluble polymer, particularly PVI or Eudragit L100-55, and Crospovidone (a water-insoluble polymer) are spray-dried by the process as described herein, to produce respective spray-dried powder blend comprising (1) said API, (2) said water-soluble polymer, (3) Crospovidone (PVPP) and (4) optionally, one or more pharmaceutical acceptable excipients, which may comprise suitable processing aids, bulking agents, plasticizer, surfactants, plasticizers, disintegrants, lubricants, glidants, carriers, anti-adherents, inert fillers, wetting agents, pH modifiers, binders, solubility modifiers, and/or recrystallization inhibitors.

The solid dispersion of the present application can be advantageously formulated with or without pharmaceutically acceptable additives. The preferred dosage formulations of the present application would include, but are not limited to, powder, granules, fine granules, tablets, rings, capsules, pellets, suppositories, ointments, plasters, cataplasms, aerosols, powders and the like.

Further, certain aspects of the present invention are illustrated in more detail by way of the below given examples. The examples are given herein for illustration of the invention and are not intended to be limiting thereof.

### Example 1 is an inventive Example, while Examples 2, 3 and 4 are reference Examples.

### Example 1: Loratadine Composition:

A loratadine solid dispersion composition comprising (a) Loratadine (b) Water-soluble polymer (copovidone) and (c) Water-insoluble polymer (crospovidone) in the ratio of **1** :1 :2 (*w*/*w*/*w*) is prepared for the present application.

### Preparation of Loratadine solid dispersion by Hot-Melt Extrusion Method

A physical mixture of the composition disclosed in Example **1** was fed through a Dynisco® laboratory mixing extruder bearing a rotor and header temperature of 130°C. The extrudate was air cooled and a portion of the material was triturated using a mortar and pestle for analytical testing.

### Characterization of solid dispersion:

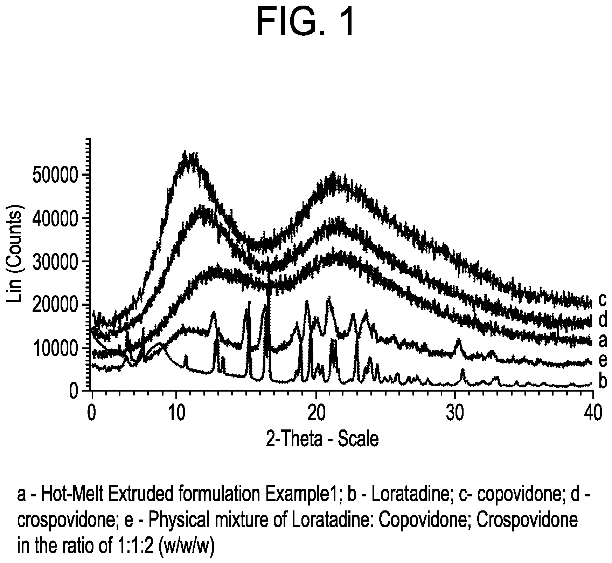

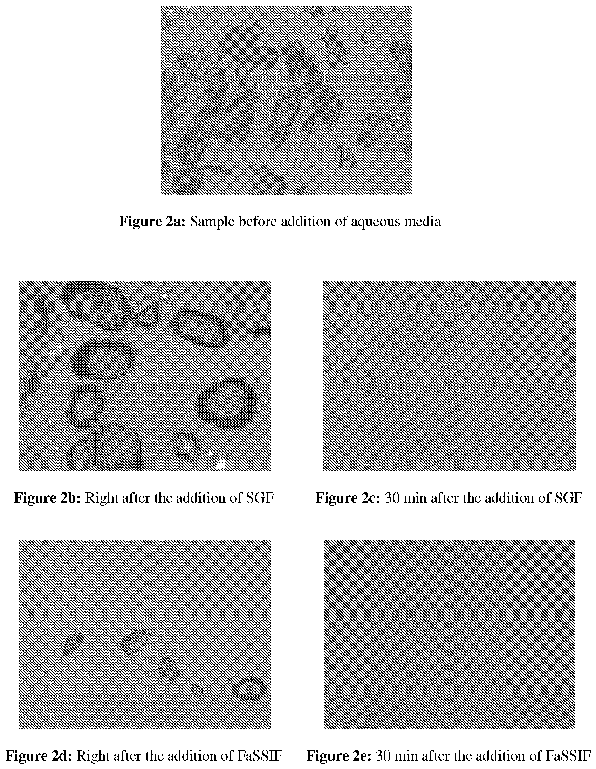

(1) X-Ray Powder Diffraction (XPRD): The following samples were evaluated by XRPD analysis (Figure 1).
   a. Hot-Melt Extruded formulation comprising formulation of Example **1**
   b. Loratadine
   c. Copovidone, a water-soluble polymer
   d. Crospovidone, a water-insoluble polymer, and
   e. Physical mixture of Loratadine: Copovidone: Crospovidone provided in the ratio of 1:1:2 (*w*/*w*/*w*).
(2) Polarized Light Microscopy (PLM): This technique was applied on the following samples to understand the crystallization potential of the desired API (Loratadine) in the presence of Simulated Gastric Fluid (SGF) and Fasted-state simulated intestinal fluid (FaSSIF). The GIT stability data of extruded formulation are disclosed in the following figures:
   a. Without addition of FaSSIF and SGF (As is) (Figure 2a)
   b. T₀ after the addition of FaSSIF and SGF (Figures 2b and 2c)
   c. T₃₀ₘᵢₙ after the addition of FaSSIF and SGF (Figures 2d and 2e)

Discussion: After extruding the composition of Example **1** (Loratadine: Copovidone: Crospovidone in the ratio 1:1:2 (w/w/w)) using the Dynisco® laboratory mixing extruder at 130°C, an amorphous solid dispersion was obtained and the formation is duly confirmed by the XRPD results. From the PLM observation, it is evident that the solid dispersion of the present application was able to prevent the re-crystallization of Loratadine in SGF and FaSSIF for at least 30 min (Figures 2a - 2d). The prevention of re-crystallization of solid dispersion of Loratadine composition suggests its potential to enhance bioavailability.

### Example 2: Lovastatin Composition:

Binary and ternary solid dispersions of Lovastatin **(LOV)** were developed with PVP K-17 and/or PVPP. The specific binary and ternary solid dispersion compositions of the present application are as follows:
2a, Binary solid dispersion of Lovastatin: PVPP (1:3), *(w*/*w)*
2b. Binary solid dispersion of Lovastatin: PVP (1:3), *(w*/*w)*
2c. Ternary solid dispersion of Lovastatin: PVPP: PVP (1:2:1), *(w*/*w*/*w)*

### Preparation of Binary/Ternary solid dispersion compositions of Lovastatin comprising PVP and/or PVPP by spray-drying method

Spray Drying was performed on a GEA SD Micro™ Spray Dryer. The feed material was atomized using a 0.5 *mm* two-fluid Schlick nozzle with the following spray drying conditions: Inlet temperature - 85°C, Outlet temperature - 55°C, Atomization pressure - 0.5 *bar,* and Atomization Flow Rate - 1.5 *kg*/*hr.* All solutions were prepared at 10% total solids in 2:1 (*w:w*) DCM (Dichloromethane): Methanol. Spray dried samples were then dried in a vacuum oven for at least 48hrs at 40°C.

### Characterization of solid dispersion:

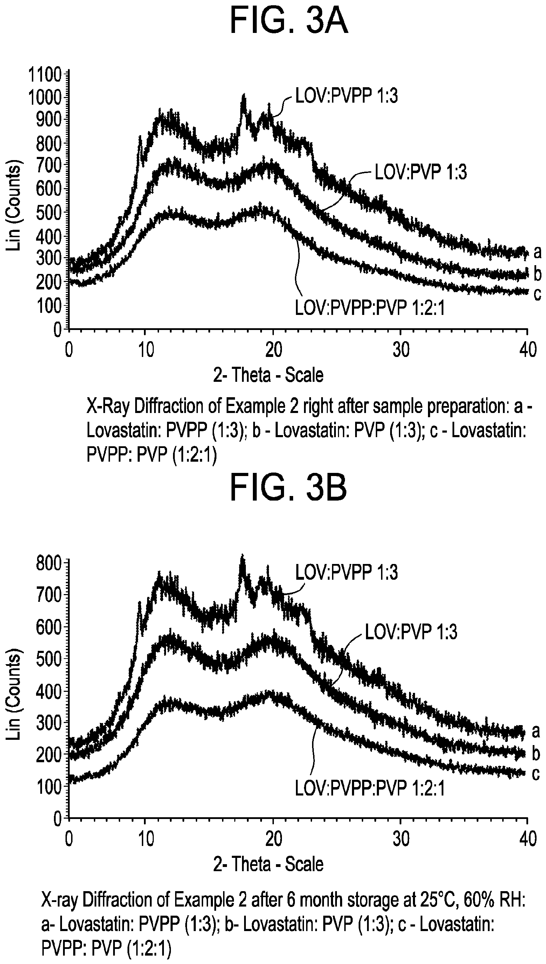

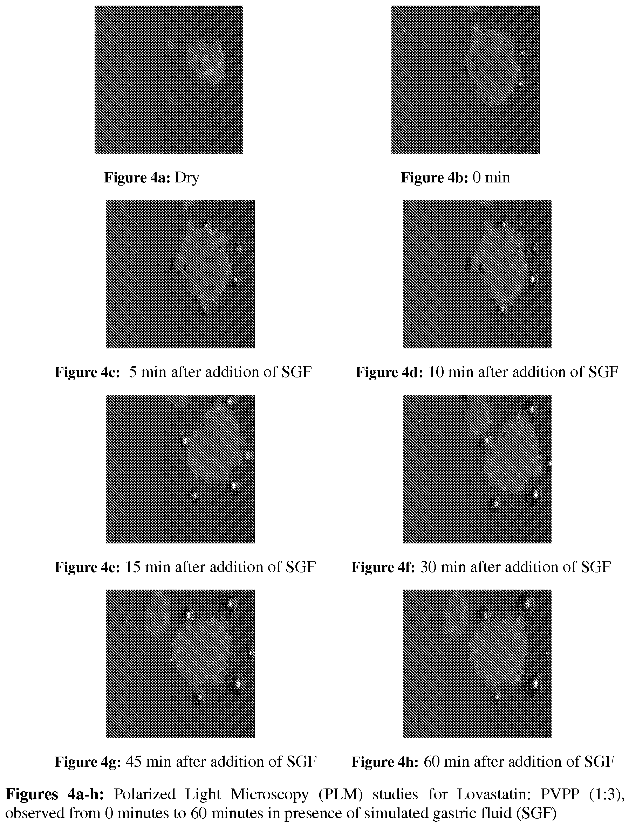

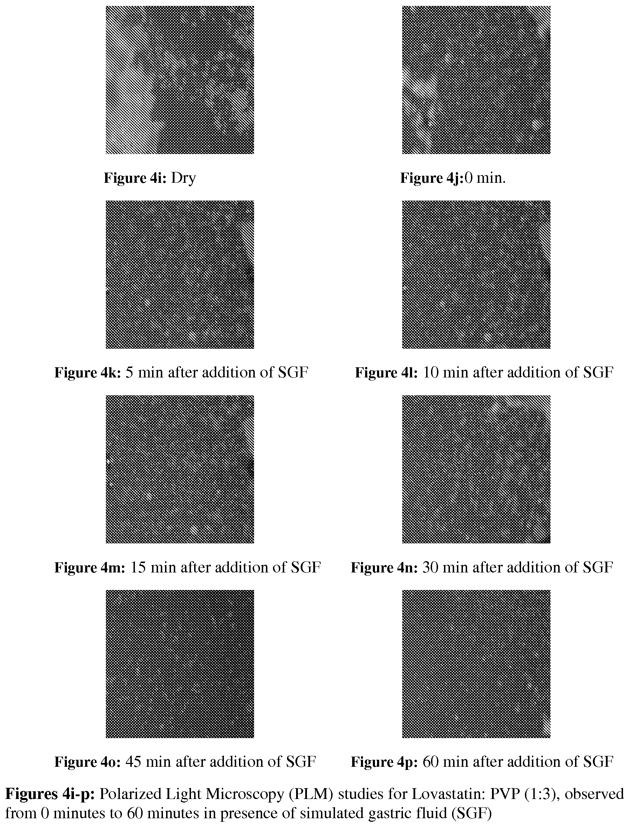

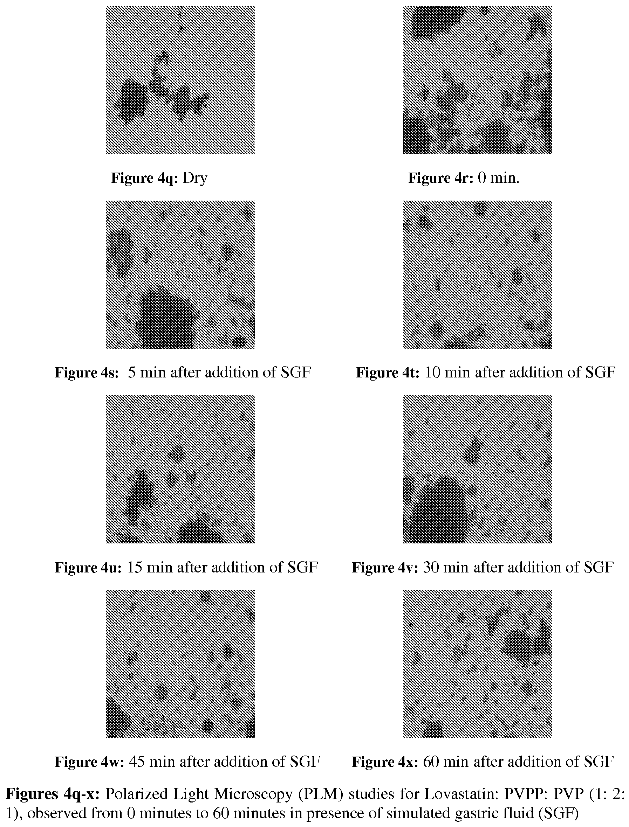

(1) X-Ray Powder Diffraction (XPRD): The X-Ray powder diffraction studies were conducted for solid dispersion compositions of Example 2a, 2b and 2c for the following two different conditions.
   a. Spray-dried formulation of Example 2a, 2b, and 2c right after sample preparation (Fig. 3a).
   b. Spray-dried formulation of Example 2a, 2b, and 2c after 6 month storage at 25°C, 60% RH (Fig. 3b).
(2) Polarized Light Microscopy (PLM):
   The PLM technique was applied to understand the crystallization potential of the desired API (Lovastatin) in the presence of Simulated Gastric Fluid (SGF). The PLM studies were conducted in three different samples including (a) Lovastatin: PVPP (1: 3), (b) Lovastatin: PVP (1:3), and (c) Lovastatin: PVPP: PVP (1:2:1) at different time intervals including 0, 5, 10, 15, 30, 45, and 60 minutes after addition of SGF. The precipitation inhibition effects of spray-dried formulation are disclosed in the following figures:
   a. Lovastatin: PVPP (1:3) (Fig. 4a-h)
   b. Lovastatin: PVP (1:3) (Fig. 4i-p)
   c. Lovastatin: PVPP: PVP (1:2:1) (Fig. 4q-x)

Discussion: After spray-drying the compositions of Examples 2a - 2c on a GEA SD Micro™ Spray Dryer with the standardized conditions, amorphous solid dispersion compositions of examples 2b and 2c were obtained as confirmed by the XPRD results (Fig. 3a). Both examples 2b and 2c remained as amorphous after 6 month storage at 25°C, 60% RH (Fig. 3b). Example 2a was a partially crystalline dispersion (Fig. 3a). From PLM observation, it is demonstrated that Lovastatin was able to form ternary amorphous solid dispersion composition with PVP and PVPP (example 2c), the prepared composition demonstrated desirable precipitation inhibition capability in SGF. Further, it was observed that the Lovastatin/PVPP binary composition was not able to form amorphous solid dispersion, and Lovastatin/PVP binary solid dispersion precipitated in SGF very rapidly. From the above experimental results, it is evident that the ternary solid dispersion composition of example 2c was able to form amorphous solid dispersion with diserable physical stability. Furthermore, the ternary solid dispersion compositions was able to prevent the crystallization of Lovastatin in SGF more efficiently than binary solid dispersions of the present application. Therefore, the ternary solid dispersion of Lovastatin composition suggests its better potential to enhance bioavailability in comparison with its relevant binary compositions.

### Example 3: Ezetimibe (EZE) Composition:

Binary and ternary solid dispersions of Ezetimibe (**EZE**) were developed with PVP K-17 and/or PVPP. The specific binary and ternary solid dispersion compositions of the present application are as follows:
3a. Binary solid dispersion of Ezetimibe: PVPP (1:3), *(w*/*w)*
3b. Binary solid dispersion of Ezetimibe: PVP (1:3), *(w*/*w)*
3c. Ternary solid dispersion of Ezetimibe: PVPP: PVP (1:2:1), *(w*/*w*/*w)*

### Preparation of Binary/Ternary solid dispersion compositions of Ezetimibe comprising PVP and/or PVPP by spray-drying method

Spray Drying was performed on a GEA SD Micro™ Spray Dryer. The feed material was atomized using a 0.5 *mm* two-fluid Schlick nozzle with the following spray drying conditions: Inlet temperature - 85°C, Outlet temperature - 55°C, Atomization pressure - 0.5 *bar,* and Atomization Flow Rate -1.5 *kg*/*hr.* All solutions were prepared at 10% total solids in 2:1 (*w:w*) DCM (Dichloromethane): Methanol. Spray dried samples were then dried in a vacuum oven for at least 48hrs at 40°C.

### Characterization of solid dispersion:

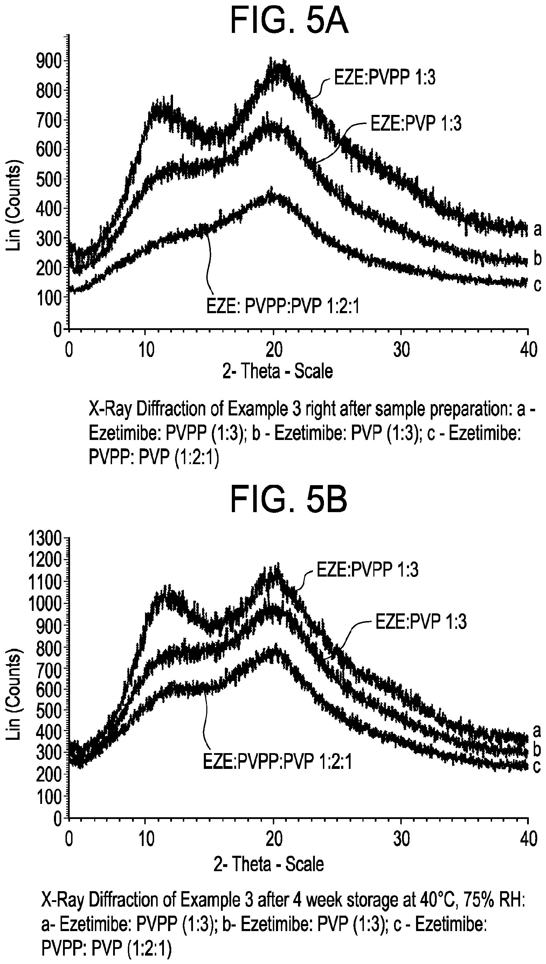

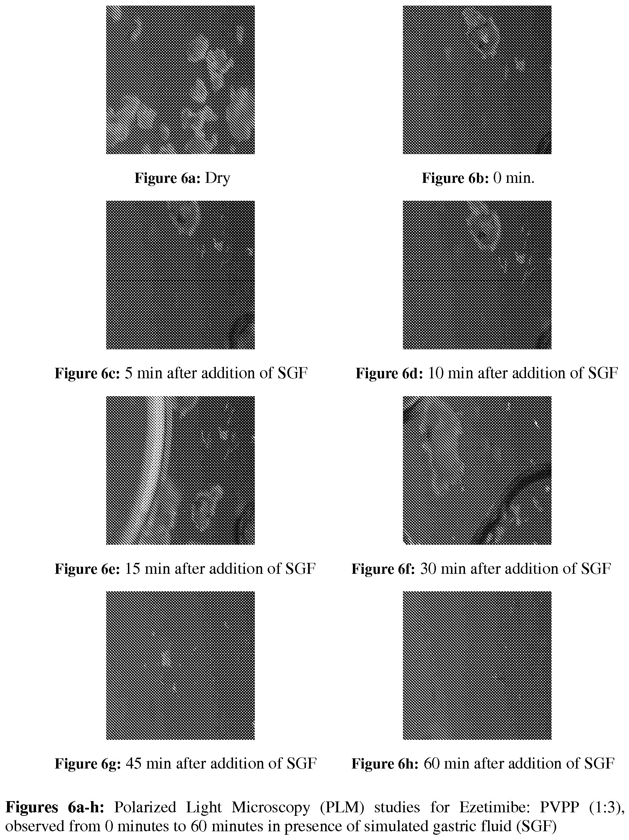

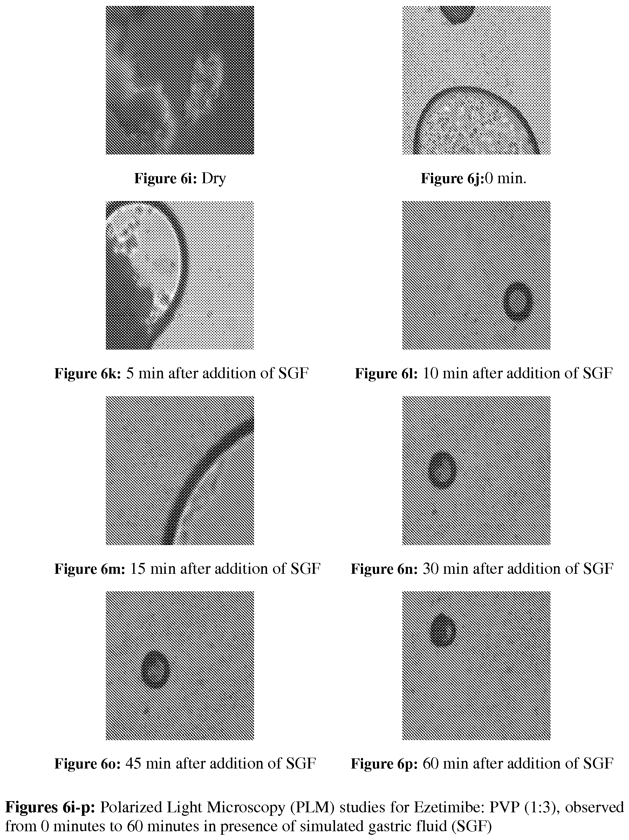

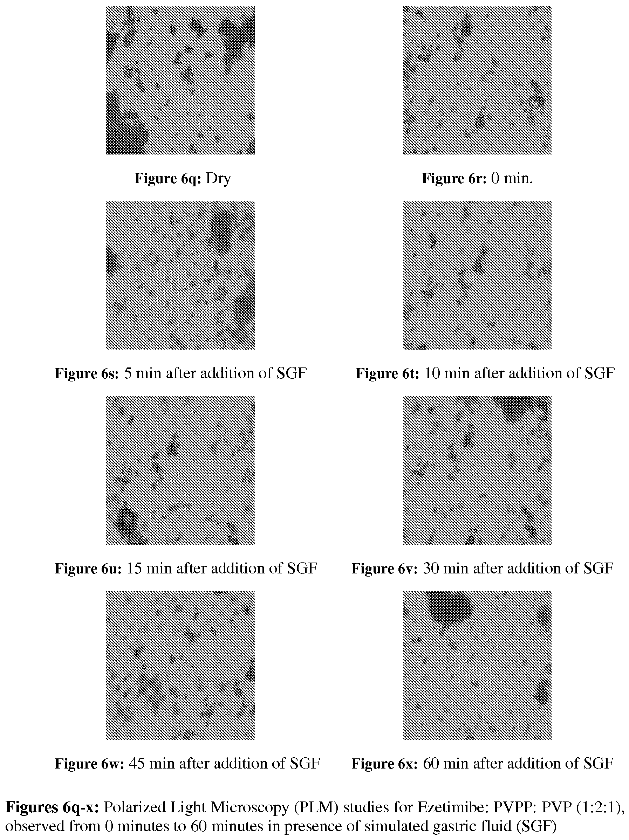

(1) X-Ray Powder Diffraction (XPRD): The X-Ray powder diffraction studies were conducted for solid dispersion compositions of Example 3a, 3b and 3c for the following two different conditions.
   a. Spray-dried formulation of Example 3a, 3b, and 3c right after sample preparation (Fig. 5a).
   b. Spray-dried formulation of Example 3a, 3b, and 3c after 4 week storage at 40°C, 75% RH (Fig. 5b).
(2) Polarized Light Microscopy (PLM):
   The PLM technique was applied to understand the crystallization potential of the desired API (Ezetimibe) in the presence of Simulated Gastric Fluid (SGF). The PLM studies were conducted in three different samples including (a) Ezetimibe: PVPP (1: 3), (b) Ezetimibe: PVP (1:3), and (c) Ezetimibe: PVPP: PVP (1 :2: 1) at different time intervals including 0, 5, 10, 15, 30, 45, and 60 minutes after addition of SGF. The GIT stability data of spray-dried formulation are disclosed in the following figures:
   a. Ezetimibe: PVPP (1:3) (Fig. 6a-h)
   b. Ezetimibe: PVP (1:3) (Fig. 6i-p)
   c. Ezetimibe: PVPP: PVP (1 :2:1) (Fig. 6q-x)
(3) Kinetic solubility test for Ezetimibe solid dispersion compositions (3a-c):
   Kinetic solubility was performed using a Pion uDISS™ Profiler. Ezetimibe samples were added to 20ml of Fasted-state simulated intestinal fluid (FaSSIF) heated to 37°C and stirred under 300 *rpm.* Each spray dried solid dispersion composition was weighed so that 1.5mg of drug was added to each vial. Dissolutions measurements were taken by *in-situ* fiber optic probes at various time points and these measurements were analyzed at a wavelength of 240*nm* for the amount of Ezetimibe dissolved.

Discussion: After spray-drying the compositions of Examples 3a-c on a GEA SD Micro™ Spray Dryer with the standardized conditions, the amorphous solid dispersion compositions of examples 3a-c were obtained as confirmed by the XPRD results (Fig. 5a). All of the examples remained as amorphous solid dispersions after 4 week storage at 40°C, 75% RH. From PLM observation, it is demonstrated that Ezetimibe was able to form ternary amorphous solid dispersion with PVP and PVPP (example 3c), the composition provides satisfactory precipitation inhibition capability in SGF (>60 min). Contrarily, it is observed that the Ezetimibe/PVPP composition precipitated in SGF very rapidly.

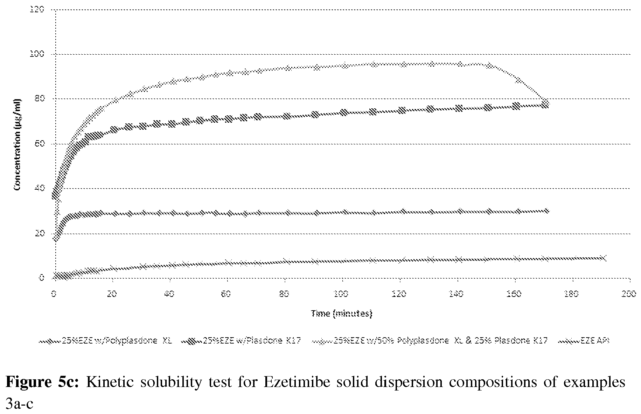

From this kinetic solubility study (Fig. 5c), the ternary solid dispersion composition of example 3c showed significantly enhanced kinetic solubility for three hours in comparison with the other two binary systems (Examples 3a-b). Thus, the ternary solid dispersion of Ezetimibe composition demonstrated its potential to better enhance bioavailability of Ezetimibe than the relevant binary compositions (Fig. 5c).

### Example 4: Piroxicam (PRX) Composition:

Binary and ternary solid dispersions of Piroxicam (PRX) were developed with Eudragit L100-55 and/or PVPP. The specific binary and ternary solid dispersion compositions of the present application are as follows:
4a. Binary solid dispersion of Piroxicam (PRX): PVPP (1:3), *(w*/*w)*
4b. Binary solid dispersion of Piroxicam (PRX): Eudragit L100-55 (1:3), (*w*/*w*)
4c. Ternary solid dispersion of Ezetimibe: PVPP: Eudragit L100-55 (1:2:1), (*w*/*w*/*w*)

### Preparation of Binary/Ternary solid dispersion compositions of Piroxicam comprising Eudragit L100-55 and/or PVPP by spray-drying method

Spray Drying was performed on a GEA SD Micro™ Spray Dryer. The feed material was atomized using a 0.5 *mm* two-fluid Schlick nozzle with the following spray drying conditions: Inlet temperature - 85°C, Outlet temperature - 55°C, Atomization pressure - 0.5 *bar,* and Atomization Flow Rate - 1.5 *kg*/*hr.* All solutions were prepared at 10% total solids in 2:1 (*w:w*) DCM (Dichloromethane): Methanol. Spray dried samples were then dried in a vacuum oven for at least 48hrs at 40°C.

### Characterization of solid dispersion:

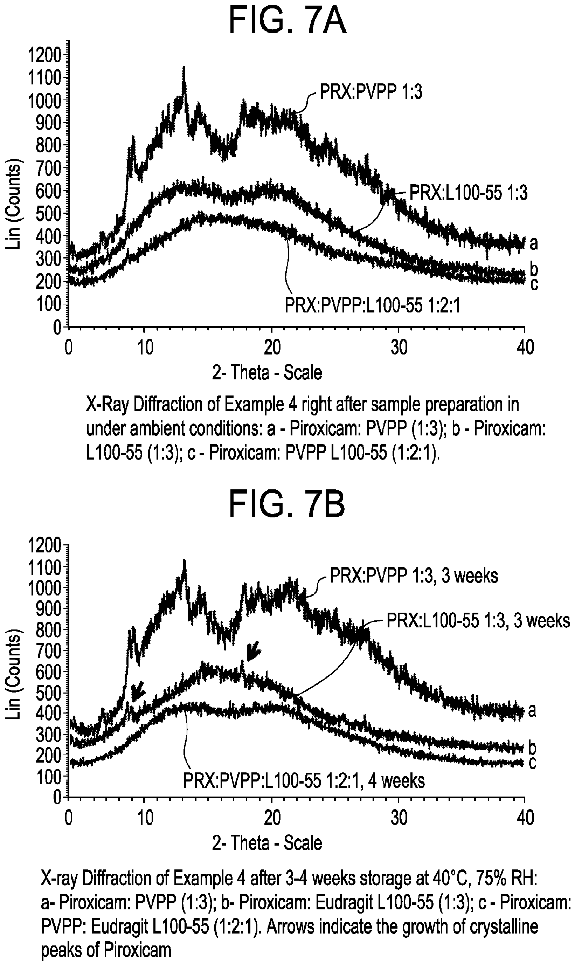

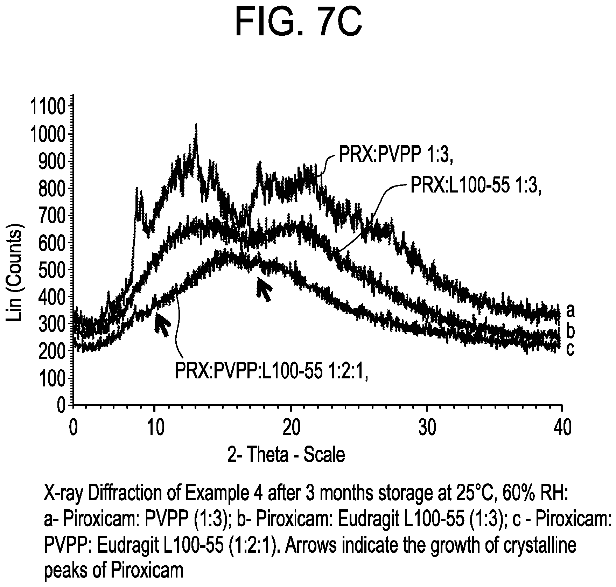

(1) X-Ray Powder Diffraction (XPRD): The X-Ray powder diffraction studies were conducted for solid dispersion compositions of Example 4a, 4b and 4c for the following two different conditions.
   a. Spray-dried formulation of Examples 4a, 4b, and 4c right after sample preparation (Fig. 7a).
   b. Spray-dried formulation of Examples 4a and 4b, after 3 weeks storage at 40°C, 75% RH, and Example 4c after 4 weeks storage at 40°C. 75% RH (Fig. 7b).
   c. Spray-dried formulation of Example 4a, 4b, and 4c after 3 months storage at 25°C, 60% RH (Fig. 7c).

Discussion: After spray-drying the compositions of Examples 4a-c on a GEA SD Micro™ Spray Dryer with the standardized conditions, amorphous solid dispersions of Examples 4b and 4c were obtained as confirmed by the XPRD results (Fig. 7a). Example 4b crystallized after 3-week storage at 40°C, 75% RH or 3-month storage at 25°C, 60% RH. Example 4c remained as amorphous solid dispersion after 4 weeks storage at 40°C, 75% RH or 3-month storage at 25°C, 60% RH. Further, it is observed that the Piroxicam/PVPP composition (Example 4a) was not able to form amorphous solid dispersion (Fig. 7a).

From the pertinent experimental results, it is observed that the binary solid dispersions were either partial crystalline or not able to remain amorphous after storage (Fig.7b and 7c). The ternary solid dispersion of Piroxicam composition (4c) demonstrated its superior physical stability in comparison with its relevant binary systems.

## Claims

1. A stable ternary solid dispersion composition prepared by hot-melt extrusion and with enhanced bioavailability comprising:
a) 15% wt. to 35% wt. of one or more poorly soluble active pharmaceutical ingredient (API) which belong to Biopharmaceutics Classification System (BCS) class II and/or IV; and
b) 15% wt. to 35% wt. of at least one water-soluble polymers; and
c) 40% wt. to 60% wt. of crosslinked polyvinylpyrrolidone;
wherein the solid dispersion is capable of inhibiting crystallization of API in the solid state and/or aqueous gastrointestinal tract (GIT) medium.

2. The solid dispersion composition according to claim 1, wherein the ratio of API (a) to water-soluble polymer (b) to crospovidone (c) is 0.5-1.5 : 0.5-1.5 : 1.5-2.5.

3. The solid dispersion composition according to claim 1, wherein the ratio of API (a) to water-soluble polymer (b) to crospovidone (c) is 1 : 1 : 2.

4. The solid dispersion composition according to claim 1, wherein said composition is storage-stable, transit-stable and/or able to increase dissolution rate and/or maintain supersaturation of API (a) during dissolution.

5. The solid dispersion composition according to claim 1, wherein said API is selected from group consisting of analgesics, anti-inflammatory agents, anti-helminthics, anti-arrhythmic agents, anti-bacterial agents, anti-viral agents, anti-coagulants, antidepressants, anti-diabetics, anti-epileptics, anti-fungal agents, anti-gout agents, antihypertensive agents, anti-malarials, anti-migraine agents, anti-muscarinic agents, antineoplastic agents, erectile dysfunction improvement agents, immune-suppressants, antiprotozoal agents, anti-thyroid agents, anxiolytic agents, sedatives, hypnotics, neuroleptics, [beta]-blockers, cardiac inotropic agents, corticosteroids, diuretics, anti-parkinsonian agents, gastrointestinal agents, histamine receptor antagonists, keratolyptics, lipid regulating agents, antianginal agents, Cox-2 inhibitors, leukotriene inhibitors, macrolides, muscle relaxants, nutritional agents, opiod analgesics, protease inhibitors, sex hormones, stimulants, muscle relaxants, anti-osteoporosis agents, anti-obesity agents, cognition enhancers, anti-urinary incontinence agents, nutritional oils, anti-benign prostate hypertrophy agents, essential fatty acids, non-essential fatty acids, antipyretics, muscular relaxants, anticonvulsants, antiemetics, anti-psychotics, anti-Alzheimer agents and combinations thereof.

6. The solid dispersion composition according to claim 1, wherein said water-soluble polymer is selected from the group consisting of homopolymers of N-vinyllactam, copolymers of N-vinyllactam, cellulose esters, cellulose ethers, polyalkylene oxides, polyacrylates, polymethacrylates, homo and co polymers of acrylic acids, homo- and co-polymers of methacrylic acids, copolyacrylamides, polyvinyl alcohols, vinyl acetate polymers, copolymers or vinylacetate, carboxyvinyl polymers, oligosaccharides, polysaccharides and mixtures thereof.

7. The solid dispersion composition according to claim 1, wherein said water-soluble polymer is selected from the group consisting of alkylcellulose, hydroxyalkylcelluloses, hydroxyalkylalkylcellulose, methylcellulose (MC), ethylcellulose (EC), hydroxyethylcellulose (HEC), hydroxypropyl cellulose (HPC), hydroxypropylmethylcellulose (HPMC), hydroxyethylmethylcellulose (HEMC), hydroxypropylmethylcellulose succinate, hydroxypropylmethyl cellulose acetate succinate, carboxymethylethylcellulose, sodium carboxymethylcellulose, pottasium carboxymethyl cellulose, cellulose acetate succinate, cellulose acetate phthalate, hydroxypropylmethylcellulose phthalate, polyacrylic acid copolymer, poly(meth)acrylic acid polymers, poly(hydroxyalkyl acrylates), poly(hydroxyalkyl methacrylates), polyvinylpyrrolidone (PVP), homopolymers of vinylpyrrolidone, copolymers of vinylpyrrolidone, povidone, vinylpyrrolidone-vinylacetate copolymer (copovidone), copolymers of vinyl acetate, copolymers of vinyl propionate, copolymers of vinyl acetate and crotonic acid, polyethylene glycol, polyvinyl alcohol, partially hydrolyzed polyvinyl acetate, gelatin, sodium alginate, soluble starch, gum acacia, dextrin, hyaluronic acid, sodium chondroitin sulfate, propylene glycol alginate, agar, tragacanth, xanthan gum, aminoalkyl methacrylate copolymers, polyvinyl-acetal-diethylaminoacetate, methacrylate copolymer, methacrylic acid copolymer L, methacrylic acid copolymer LD, methacrylic acid copolymer S, macrogol, polyethylene oxide, polypropylene oxide, copolymers of ethylene oxide (EO) and propylene oxide (PO), carrageenans, galactomannans and mixtures thereof.

8. The solid dispersion composition according to claim 1 further comprising an anionic, cationic, non-ionic or amphoteric surfactant present in an amount of from 0.001 % wt. to 5.0% wt. of the total composition, wherein said surfactant is preferably selected from the group consisting of dodecanesulfonic acid, sodium dodecyl sulfate, sodium lauryl sulfate (SLS), (poly)-oxyethylene sorbitan long-chain fatty acid esters, Vitamin E-TPGS, bile salts, sodium deoxycholate, sodium glycocholate, polyoxyethylene polyoxypropylene glycols and combinations thereof.

9. The solid dispersion composition according to claim 1 further comprising a plasticizer present in an amount of from 0.1 % wt. to 10.0% wt. of the total composition, wherein said plasticizer is preferably selected from the group consisting of Triethyl Citrate, Glycerol Monostearate, Dibutyl Sebacate, Diethyl Phthalate, Polyethylene Glycol, Triacetin, Vitamin E-TPGS, Tween 80, Sodium Lauryl Sulfate, Sodium Docusate, Poloxamer F-68, Poloxamer F-127 and combinations thereof.

10. The solid dispersion composition according to claim 1 formulated with pharmaceutically acceptable excipients selected from the group consisting of disintegrants, lubricants, glidants, carriers, anti-adherents, inert fillers, wetting agents, pH modifiers, binders, solubility modifiers, recrystallization inhibitors, diluents and combinations thereof.

11. The solid dispersion composition according to claim 1, wherein said solid dispersion is formulated into tablets, rings, patches, capsules, pellets, granules, fine granules or a powder.

12. A method for preparing the solid dispersion composition according to claim 1 comprising the steps of:
a. preparing a homogenous blend of (i) at least one active pharmaceutical ingredient (API) which belongs to BCS class II and/or IV; (ii) one or more water-soluble polymer; and (iii) crosslinked polyvinylpyrrolidone;
b. heating, mixing and kneading the resultant blend of step (a) via an extruder to result in a homogenous melt;
c. forcing the resultant melt obtained in step (b) through one or more orifices, nozzles, or moulds;
d. cooling the extrudate of step (c) by means of air to yield a solid dispersion; and
e. optionally, grinding the solid dispersion obtained in step (d).

## Patentansprüche

1. Eine stabile ternäre feste Dispersionszusammensetzung, die durch Heißschmelzextrusion hergestellt wird und eine verbesserte Bioverfügbarkeit aufweist, umfassend:
a) 15 Gew.-% bis 35 Gew.-% eines oder mehrerer schwer löslicher pharmazeutischer Wirkstoffe (API), die zur Klasse II und/oder IV des Biopharmazeutischen Klassifizierungssystems (BCS) gehören; und
b) 15 Gew.-% bis 35 Gew.-% mindestens eines wasserlöslichen Polymers; und
c) 40 Gew.-% bis 60 Gew.-% vernetztes Polyvinylpyrrolidon;
wobei die feste Dispersion in der Lage ist, die Kristallisation des API im festen Zustand und/oder im wässrigen Medium des Gastrointestinaltraktes (GIT) zu hemmen.

2. Die feste Dispersionszusammensetzung gemäß Anspruch 1, wobei das Verhältnis des API (a) zu wasserlöslichem Polymer (b) zu Crospovidon (c) 0,5-1,5: 0,5-1,5: 1,5-2,5 beträgt.

3. Die feste Dispersionszusammensetzung gemäß Anspruch 1, wobei das Verhältnis des API (a) zu wasserlöslichem Polymer (b) zu Crospovidon (c) 1 : 1 : 2 beträgt.

4. Die feste Dispersionszusammensetzung gemäß Anspruch 1, wobei die Zusammensetzung lagerstabil, transportstabil und/oder in der Lage ist, die Auflösungsrate zu erhöhen und/oder die Übersättigung des API (a) während der Auflösung aufrechtzuerhalten.

5. Die feste Dispersionszusammensetzung gemäß Anspruch 1, wobei der Wirkstoff ausgewählt wird aus der Gruppe bestehend aus Analgetika, entzündungshemmenden Mitteln, Antihelminthika, antiarrhythmischen Mitteln, antibakteriellen Mitteln, antiviralen Mitteln, Antikoagulantien, Antidepressiva, Antidiabetika, Antiepileptika, Antimykotika, Antigichtmitteln, Antihypertonika, Anti-Malariamittel, Anti-Migräne-Mittel, Anti-Muskarinika, antineoplastische Mittel, Mittel zur Verbesserung der erektilen Dysfunktion, Immunsuppressiva, Antiprotozoenmittel, Anti-Schilddrüsenmittel, Anxiolytika, Sedativa, Hypnotika, Neuroleptika, [Beta]-Blocker, kardiale Inotropika, Kortikosteroide, Diuretika, Anti-Parkinson-Wirkstoffe, Magen-Darm-Wirkstoffe, Histaminrezeptor-Antagonisten, Keratolyptika, Lipidregulatoren, Antiangin-Wirkstoffe, Cox-2-Inhibitoren, Leukotrien-Inhibitoren, Makrolide, Muskelrelaxantien, Nahrungsergänzungsmittel, Opioid-Analgetika, Protease-Inhibitoren, Sexualhormone, Stimulanzien, Muskelrelaxantien, Anti-Osteoporose-Wirkstoffe, Anti-Adipositas-Wirkstoffe, Kognitionsförderer, Mittel gegen Harninkontinenz, Nahrungsöle, Mittel gegen Prostatahypertrophie, essentielle Fettsäuren, nicht-essentielle Fettsäuren, Anti-Fiebersubstanzen, Muskelrelaxantien, Anti-Konvulsiva, Antiemetika, Anti-Psychopharmaka, Anti-Alzheimer-Wirkstoffe und Kombinationen davon.

6. Die feste Dispersionszusammensetzung gemäß Anspruch 1, wobei das wasserlösliche Polymer ausgewählt wird aus der Gruppe bestehend aus Homopolymeren von N-Vinyllactam, Copolymeren von N-Vinyllactam, Celluloseestern, Celluloseethern, Polyalkylenoxiden, Polyacrylaten, Polymethacrylate, Homo- und Copolymere von Acrylsäuren, Homo- und Copolymere von Methacrylsäuren, Copolyacrylamide, Polyvinylalkohole, Vinylacetatpolymere, Copolymere oder Vinylacetat, Carboxyvinylpolymere, Oligosaccharide, Polysaccharide und Mischungen davon.

7. Die feste Dispersionszusammensetzung gemäß Anspruch 1, wobei das wasserlösliche Polymer ausgewählt wird aus der Gruppe bestehend aus Alkylcellulose, Hydroxyalkylcellulosen, Hydroxyalkylalkylcellulose, Methylcellulose (MC), Ethylcellulose (EC), Hydroxyethylcellulose (HEC), Hydroxypropylcellulose (HPC), Hydroxypropylmethylcellulose (HPMC), Hydroxyethylmethylcellulose (HEMC), Hydroxypropylmethylcellulose-Succinat, Hydroxypropylmethylcelluloseacetatsuccinat, Carboxymethylethylcellulose, Natriumcarboxymethylcellulose, Kaliumcarboxymethylcellulose, Celluloseacetatsuccinat, Celluloseacetatphthalat, Hydroxypropylmethylcellulosephthalat, Polyacrylsäure-Copolymer, Poly(meth)-acrylsäure-Polymere, Poly(hydroxyalkylacrylate), Poly(hydroxyalkylmethacrylate), Polyvinylpyrrolidon (PVP), Homopolymere von Vinylpyrrolidon, Copolymere von Vinylpyrrolidon, Povidon, Vinylpyrrolidon-Vinylacetat-Copolymer (Copovidon), Copolymere von Vinylacetat, Copolymere von Vinylpropionat, Copolymere aus Vinylacetat und Crotonsäure, Polyethylenglykol, Polyvinylalkohol, teilweise hydrolysiertes Polyvinylacetat, Gelatine, Natriumalginat, lösliche Stärke, Gummi arabicum, Dextrin, Hyaluronsäure, Natriumchondroitinsulfat, Propylenglykolalginat, Agar, Traganth, Xanthangummi, Aminoalkylmethacrylat-Copolymere, Polyvinylacetaldiethylaminoacetat, Methacrylat-Copolymer, Methacrylsäure-Copolymer L, Methacrylsäure-Copolymer LD, Methacrylsäure-Copolymer S, Makrogol, Polyethylenoxid, Polypropylenoxid, Copolymere von Ethylenoxid (EO) und Propylenoxid (PO), Carrageene, Galactomannane und Mischungen davon.

8. Die feste Dispersionszusammensetzung gemäß Anspruch 1, die weiterhin ein anionisches, kationisches, nichtionisches oder amphoteres Tensid umfasst, das in einer Menge von 0,001 Gew.-% bis 5,0 Gew.-% der Gesamtzusammensetzung vorhanden ist, wobei das Tensid vorzugsweise aus der Gruppe ausgewählt wird, die aus Dodecansulfonsäure, Natriumdodecylsulfat, Natriumlaurylsulfat (SLS), (Poly)-oxyethylensorbitan-langkettigen Fettsäureestern, Vitamin E-TPGS, Gallensalzen, Natriumdesoxycholat, Natriumglycocholat, Polyoxyethylenpolyoxypropylenglykolen und Kombinationen davon besteht.

9. Die feste Dispersionszusammensetzung gemäß Anspruch 1, die weiterhin einen Weichmacher umfasst, der in einer Menge von 0,1 Gew.-% bis 10,0 Gew.-% der Gesamtzusammensetzung vorhanden ist, wobei der Weichmacher vorzugsweise aus der Gruppe ausgewählt wird, die aus Triethylcitrat, Glycerinmonostearat, Dibutylsebacat, Diethylphthalat, Polyethylenglykol, Triacetin, Vitamin E-TPGS, Tween 80, Natriumlaurylsulfat, Natriumdocusat, Poloxamer F-68, Poloxamer F-127 und Kombinationen davon besteht.

10. Die feste Dispersionszusammensetzung gemäß Anspruch 1, die mit pharmazeutisch verträglichen Hilfsstoffen, ausgewählt aus der Gruppe bestehend aus Sprengmitteln, Schmiermitteln, Gleitmitteln, Trägern, Antihaftmitteln, inerten Füllstoffen, Netzmitteln, pH-Modifikatoren, Bindemitteln, Löslichkeitsmodifikatoren, Rekristallisationsinhibitoren, Verdünnungsmitteln und Kombinationen davon formuliert ist.

11. Die feste Dispersionszusammensetzung gemäß Anspruch 1, wobei die feste Dispersion zu Tabletten, Ringen, Pflastern, Kapseln, Pellets, Körnern, feinen Körnern oder einem Pulver formuliert ist.

12. Verfahren zur Herstellung der festen Dispersionszusammensetzung gemäß Anspruch 1, umfassend die folgenden Schritte
a. Herstellen einer homogenen Mischung aus (i) mindestens einem aktiven pharmazeutischen Inhaltsstoff (API), der zur BCS-Klasse II und/oder IV gehört; (ii) einem oder mehreren wasserlöslichen Polymeren; und (iii) vernetztem Polyvinylpyrrolidon;
b. Erhitzen, Mischen und Kneten der resultierenden Mischung aus Schritt (a) über einen Extruder, um eine homogene Schmelze zu erhalten;
c. Zwingen der in Schritt (b) erhaltenen Schmelze durch eine oder mehrere Öffnungen, Düsen oder Formen;
d. Kühlen des Extrudats aus Schritt (c) mittels Luft, um eine feste Dispersion zu erhalten; und
e. gegebenenfalls Mahlen der in Schritt (d) erhaltenen festen Dispersion.

## Revendications

1. Composition de dispersion solide ternaire stable préparée par extrusion à chaud et comportant une biodisponibilité augmentée comprenant :
a) 15 % en poids à 35 % en poids d'un ou plusieurs ingrédients pharmaceutiquement actifs (API) faiblement solubles qui appartiennent à la classe II et/ou IV du Système de Classification des produits Biopharmaceutiques (SCB) ; et
b) 15 % en poids à 35 % en poids d'au moins un polymère soluble dans l'eau ; et
c) 40 % en poids à 60 % en poids de polyvinylpyrrolidone réticulée ;
la dispersion solide étant capable d'inhiber la cristallisation d'un API dans l'état solide et/ou dans un milieu aqueux de tract gastro-intestinal (TGI) .

2. Composition de dispersion solide selon la revendication 1, le rapport d'API (a) à polymère soluble dans l'eau (b) à crospovidone (c) étant de 0,5 à 1,5:0,5 à 1,5:1,5 à 2,5.

3. Composition de dispersion solide selon la revendication 1, le rapport d'API (a) à polymère soluble dans l'eau (b) à crospovidone (c) étant de 1:1:2.

4. Composition de dispersion solide selon la revendication 1, ladite composition étant stable au stockage, stable au transit et/ou capable d'augmenter la vitesse de dissolution et/ou maintenant la supersaturation d'API (a) pendant la dissolution.

5. Composition de dispersion solide selon la revendication 1, ledit API étant choisi dans le groupe constitué par les analgésiques, les agents anti-inflammatoires, les anti-helminthiques, les agents anti-arythmiques, les agents antibactériens, les agents antiviraux, les anticoagulants, les antidépresseurs, les antidiabétiques, les antiépileptiques, les agents antifongiques, les agents anti-goutte, les agents antihypertenseurs, les antipaludiques, les agents anti-migraine, les agents anti-muscariniques, les agents antinéoplasiques, les agents d'amélioration du dysfonctionnement érectile, les immunosuppresseurs, les agents anti-protozoaires, les agents antithyroïdiens, les agents anxiolytiques, les sédatifs, les hypnotiques, les neuroleptiques, les bêtabloquants, les agents inotropes cardiaques, les corticostéroïdes, les diurétiques, les agents antiparkinsoniens, les agents gastro-intestinaux, les antagonistes du récepteur de l'histamine, les kératolytiques, les agents de régulation des lipides, les agents antiangineux, les inhibiteurs de Cox-2, les inhibiteurs de leucotriènes, les macrolides, les relaxants musculaires, les agents nutritionnels, les analgésiques opioïdes, les inhibiteurs de protéase, les hormones sexuelles, les stimulants, les relaxants musculaires, les agents anti-ostéoporose, les agents anti-obésité, les rehausseurs de la cognition, les agents anti-incontinence urinaire, les huiles nutritionnelles, les agents anti-hypertrophie de la prostate bénigne, les acides gras essentiels, les acides gras non essentiels, les antipyrétiques, les relaxants musculaires, les anticonvulsants, les antiémétiques, les antipsychotiques, les agents anti-Alzheimer et des combinaisons correspondantes.

6. Composition de dispersion solide selon la revendication 1, ledit polymère soluble dans l'eau étant choisi dans le groupe constitué par les homopolymères de N-vinyllactame, les copolymères de N-vinyllactame, les esters de cellulose, les éthers de cellulose, les poly(oxydes d'alkylène), les polyacrylates, les polyméthacrylate, les homopolymères et les copolymères d'acides acryliques, les homopolymères et les copolymères d'acides méthacryliques, les copolyacrylamides, les poly(alcools vinyliques), les polymères d'acétate de vinyle, les copolymères d'acétate de vinyle, les polymères de carboxyvinyle, les oligosaccharides, les polysaccharides et des mélanges correspondants.

7. Composition de dispersion solide selon la revendication 1, ledit polymère soluble dans l'eau étant choisi dans le groupe constitué par une alkylcellulose, une hydroxyalkylcellulose, une hydroxyalkylalkylcellulose, la méthylcellulose (MC), l'éthylcellulose (EC), l'hydroxyéthylcellulose (HEC), l'hydroxypropylcellulose (HPC), l'hydroxypropylméthylcellulose (HPMC), l'hydroxyéthylméthylcellulose (HEMC), le succinate d'hydroxypropylméthylcellulose, l'acétate succinate d'hydroxypropylméthylcellulose, la carboxyméthyléthylcellulose, la carboxyméthylcellulose sodique, la carboxyméthylcellulose potassique, l'acétate succinate de cellulose, l'acétate phtalate de cellulose, le phtalate d'hydroxypropylméthylcellulose, un copolymère de poly(acide acrylique), les polymères de poly(acide (méth)acrylique), les poly(acrylates d'hydroxyalkyle), les poly(méthacrylates d'hydroxyalkyle), la polyvinylpyrrolidone (PVP), les homopolymères de vinylpyrrolidone, les copolymères de vinylpyrrolidone, la povidone, un copolymère vinylpyrrolidone-acétate de vinyle (copovidone), les copolymères d'acétate de vinyle, les copolymères de propionate de vinyle, les copolymères d'acétate de vinyle et d'acide crotonique, un polyéthylèneglycol, un poly(alcool vinylique), un poly(acétate de vinyle) partiellement hydrolysé, la gélatine, un alginate de sodium, un amidon soluble, la gomme d'acacia, la dextrine, l'acide hyaluronique, le sulfate de chondroïtine sodique, un alginate de propylèneglycol, l'agar, le tragacanthe, la gomme de xanthane, les copolymères de méthacrylate d'aminoalkyle, le polyvinyl-acétal-diéthylaminoacétate, un copolymère de méthacrylate, un copolymère d'acide méthacrylique L, un copolymère d'acide méthacrylique LD, un copolymère d'acide méthacrylique S, un macrogol, un poly(oxyde éthylène), un poly(oxyde de propylène), les copolymères d'oxyde d'éthylène (EO) et d'oxyde de propylène (PO), les carraghénanes, les galactomannanes et des mélanges correspondants.

8. Composition de dispersion solide selon la revendication 1 comprenant en outre un tensioactif anionique, cationique, non ionique ou amphotère présent en une quantité allant de 0,001 % en poids à 5,0 % en poids de la composition totale, ledit tensioactif étant préférablement choisi dans le groupe constitué par l'acide dodécanesulfonique, le dodécylsulfate de sodium, le laurylsulfate de sodium (SLS), des esters d'acides gras à longue chaîne de polyoxyéthylène sorbitane, la vitamine E-TPGS, les sels biliaires, le désoxycholate de sodium, le glycocholate de sodium, les polyoxyéthylène polyoxypropylène glycols et des combinaisons correspondantes.

9. Composition de dispersion solide selon la revendication 1 comprenant en outre un plastifiant présent en une quantité allant de 0,1 % en poids à 10,0 % en poids de la composition totale, ledit plastifiant étant préférablement choisi dans le groupe constitué par le citrate de triéthyle, le monostéarate de glycérol, le sébaçate de dibutyle, le phtalate de diéthyle, un polyéthylèneglycol, la triacétine, la vitamine E-TPGS, le Tween 80, le laurylsulfate de sodium, le docusate de sodium, le poloxamère F-68, le poloxamère F-127 et des combinaisons correspondantes.

10. Composition de dispersion solide selon la revendication 1 formulée avec des excipients pharmaceutiquement acceptables choisis dans le groupe constitué par les désintégrants, les lubrifiants, les agents glissants, les supports, les agents anti-adhérence, les charges inertes, les agents mouillants, les modificateurs de pH, les liants, les modificateurs de solubilité, les inhibiteurs de recristallisation, les diluants et des combinaisons correspondantes.

11. Composition de dispersion solide selon la revendication 1, ladite dispersion solide étant formulée en comprimés, en anneaux, en patchs, en capsules, en pastilles, en granulés, en granulés fins ou en une poudre.

12. Procédé pour la préparation de la composition de dispersion solide selon la revendication 1 comprenant les étapes de :
a. préparation d'un assemblage homogène de (i) au moins un ingrédient pharmaceutiquement actif (API) qui appartient à la classe II et/ou IV du SCB ; (ii) un ou plusieurs polymères solubles dans l'eau ; et (iii) une polyvinylpyrrolidone réticulée ;
b. chauffage, mélange et malaxage de l'assemblage résultant de l'étape (a) via une extrudeuse pour donner une masse fondue homogène ;
c. forçage de la masse fondue résultante obtenue dans l'étape (b) à travers un(e) ou plusieurs orifices, buses, ou moules ;
d. refroidissement de l'extrudat de l'étape (c) au moyen d'air pour fournir une dispersion solide ; et
e. éventuellement, broyage de la dispersion solide obtenue dans l'étape (d).
